# EUROPEAN PATENT APPLICATION

(11) **EP 4 675 277 A1**
(43) Date of publication of application: **07.01.2026**
(21) Application number: 24186837.1
(22) Date of filing: 05.07.2024
(51) Int. Cl.: G01N 33/68

(54) **A METHOD FOR DIAGNOSING ALZHEIMER´S DISEASE OR DETERMINING THE RISK OF SUFFERING FROM ALZHEIMER´S DISEASE**

(71) Applicant: Predemtec AG, 9200 Gossau (CH)
(72) Inventor: The designation of the inventor has not yet been filed
(74) Representative: Kilger, Ute

(57) **Abstract**

The present invention relates to a method for diagnosing or determining the risk of suffering from Alzheimer's disease in a subject, wherein the method comprises determining the level of at least the biomarkers Neurofilament light Chain (NfL), brain-derived neurotrophic factor (BDNF), and tumor growth factor beta 1 (TGF-beta 1) and in addition determining the level of one or more biomarkers selected from the group comprising interleukin 18 (IL-18), Monocyte chemotactic protein-1 (MCP-1), Insulin-like growth factor (IGF) and Vascular endothelial growth factor (VEGF) in a sample of bodily fluid of said subject, calculating a score from the determined biomarker levels, and comparing said score with a reference score, and wherein said subject is diagnosed with Alzheimer's disease, or said subject is determined as having a risk of suffering from Alzheimer's disease, if the score is above said reference score.

## Description

Subject matter of the invention is a method for diagnosing Alzheimer's disease or determining the risk of suffering from Alzheimer's disease in a subject. Said method comprises determining the level of at least the biomarkers Neurofilament light Chain (NfL), brain-derived neurotrophic factor (BDNF), and tumor growth factor beta 1 (TGF-beta 1) and in addition determining the level of one or more biomarkers selected from the group comprising interleukin 18 (IL-18), Monocyte chemotactic protein-1 (MCP-1), Insulin-like growth factor (IGF) and Vascular endothelial growth factor (VEGF) in a sample of bodily fluid of said subject, calculating a score from the determined biomarker levels, comparing said score with a reference score. A subject is diagnosed with Alzheimer's disease or is determined as having a risk of suffering from Alzheimer's disease, if the score is above said reference score.

### Background

Millions of people worldwide are affected by neurodegenerative diseases that can lead to dementia (Alzheimer's Disease International (ADI), Wimo A, Prince M, International AD. World Alzheimer Report 2015, The Global Impact of Dementia. Alzheimer's Dis. Int. (ADI ) (2015)), first and foremost by Alzheimer's Disease (AD). This has large-scale social and economic implications and poses a heavy burden individually for the patients themselves and their caretakers, as well as for the whole community in the form of finance needed for diagnostics and treatment of the disease. Thus, there is a need for an easily accessible, minimally invasive, economical, and reliable diagnostic test capable of validly detecting the presence of AD earlier than is currently the case. According to a survey conducted by the 2019 World Alzheimer Report (Alzheimer's Disease International. World Alzheimer Report 2019: Attitudes to dementia, (2019)), 95% of around 70,000 respondents think, they could develop dementia in their lifetime, and 82% would take a genetic test to learn about their risk. Being aware early of having an elevated risk for developing dementia due to AD and subsequently making lifestyle changes can help to delay cognitive worsening in subjects at risk for dementia (Kivipelto M, et al. Nat. Rev. Neurol. 14(11), 653-666 (2018)). With this there is evidence showing the positive and lasting effect of primary and secondary prevention in AD (Hsu D, A. et al. Curr. Alzheimer Res. 14(4), 426-440 (2017)). By taking preventive measures, there could be several millions of fewer patients and their caregivers burdened by the disease (Brookmeyer R, et al. Alzheimer's Dement. 3(3), 186-191 (2007)).

Furthermore, accurate diagnostic measures in general are in demand not only by the wider public but also by pharmaceutical companies for various other reasons. So far, very limited therapies against AD are available, but the hunt for an adequate curative treatment is continuing. Due to the not fully understood etiology and reason for the heterogeneity of AD, a wider range of biomarkers might help to determine the individual risk and nature of a patient's disease.

In the past 20 years, there have been great advances to identify suitable biomarkers to detect AD, especially in early pathological stages, when the disease does not reveal clear clinical symptoms yet. The first attempt was to establish AD biomarkers traceable in cerebrospinal fluid (CSF) (see for example Hampel et al. Alzheimers Dement. 2008 Jan; 4(1):38-48; Cavedo et al. J Prev Alzheimers Dis. 2014 Dec; 1(3): 181-202).

This has been currently superseded by the search for AD biomarkers, which can be reliably identified in blood (see for example Thambisetty et al. Blood-based biomarkers of Alzheimer's disease: challenging but feasible. Biomark Med. 2010 Feb; 4(1):65-79; Hampel et al. Nat Rev Neurol. 2018 Nov; 14(11):639-652). The focus on blood-based biomarkers allows a far lower degree of invasiveness. They therefore also have a wider field of application, such as in primary screenings. No doubt also, that they will have a greater acceptance among patients.

Still, the analysis of CSF-related biomarkers in blood remains challenging. The concentration of a CSF-related biomarker released into the blood will be less concentrated and most probably, there will be interfering proteolytic processes, which may hamper analytic processes. Thus, high-resolution analytic methods are requested.

Single biomarkers have already shown to be promising in blood. Biomarkers for phosphorylated tau, neurofilament light or amyloid beta biomarker levels or ratios (Abeta42 or Abeta 42/40) have been alleged to be a helpful screening tool in different clinical settings (Ashton, N.J., Hye, A., Rajkumar, A.P. et al. Nat Rev Neurol 16, 265-284 (2020)).

However, science in this field still is in the search mode. Studies are conducted using single biomarkers or combinations of two biomarkers (such as in Chiu MJ et al. ACS Chem Neurosci. 2013 Dec 18;4(12):1530-6). The cohorts are still rather small (Henkel, A.W., Müller, K., Lewczuk, P. et al. J Neural Transm 119, 779-788 (2012); Sugerman et al. (2020) Neurobiology of Aging. 94. 10.1016).

Moreover, participants in the studies often pass several preselection steps. They are often recruited from long term studies and have thus passed various diagnostic procedures before being selected for a biomarker analysis study. Besides social, family and medical history, these diagnostic procedures comprise cerebral computer tomography and neuropsychological testings such as the Mini-Mental State Examination (MMSE), 30-item short form Boston Naming Test or Wechsler Adult Intelligence Scale IV Test (Weintraub et al. Alzheimer Dis Assoc Disord. 2009 Apr-Jun;23(2):91-101).

They also often comprise a biomarker pre-screening in CSF-samples, selecting patients that, for example, show decreased levels of CSF Abeta 42/40 (Del Prete et al. Fluid Candidate Biomarkers for Alzheimer's Disease: A Precision Medicine Approach. J. Pers. Med. 2020, 10, 221.)

Thus, patients who are selected for biomarker clinical trials have been submitted to several pre-analytic procedures before. This certainly leads to considerable progresses in the development of suitable AD biomarkers, however, makes it difficult to compare study results, especially in view of statistical accuracy (O'Bryant et al. Comparing biological biomarkers of Alzheimer's disease across blood fraction and platforms: Comparing apples to oranges. https://www.sciencedirect.com/science/journal/23528729, Vol. 3, 2016, 27-34). And, not least, diagnosis is limited to highly specialized clinical centers, which are able to provide all these disciplines.

Science is thus still far from providing a practicable test, which clearly classifies a patient into AD or non-AD, especially before the onset of clearly observable clinical symptoms. A test, which could be provided at the general practitioner in order to give a first assessment for a patient presented with memory problems, is highly desirable.

Moreover, there is a strong need to develop blood-based biomarker tests, which provide highly reliable results, which are methodologically standardized and thus may finally help to reduce pre-analytic diagnostics to a minimum.

Furthermore, a blood-based test, which allows early detection of AD or of the risk of suffering from AD would help to earlier identify and choose effective treatment for a patient. Especially in early stages, when Alzheimer does not reveal any major and distinct clinical symptoms, but neurodegeneration already occurs, preventive therapies could be started earlier and thus with a more promising outcome.

AD is a multifactorial disease. The neurodegenerative aspect of AD has been known for long already. Biomarkers related to disturbed amyloid metabolism and neuronal death have been identified, such as amyloid beta. Amyloid beta peptides aggregate and form plaques in the brain. It thus tightly correlates with the early development of AD. However, the development of reliable biomarkers has been challenging, especially due to the property of the Amyloid peptides to self-aggregate or bind to other proteins. Nevertheless, the ratio of the amyloid beta biomarkers Abeta 42 and Abeta 40, has been shown to be a reliable biomarker for AD in CSF (Hampel et al. Exp Neurol. 2010 Jun;223(2):334-46).

In a diagnostics context, molecules in biofluids reflecting these processes might be biomarker candidates for AD. However, there are several challenges in developing a reliable and valid test to detect AD-typical changes in peripheral biofluids such as blood (for an overview see Menne et al., 2021). Reasons for contradictory evidence might be differing material for blood drawing and processing, like stoppers or separator gels in blood tubes, that were shown to influence test results (López-Bascón MA, et al. Talanta. 2016. doi:10.1016/j.talanta.2015.12.079 and De Jager W, et al. BMC Immunol. 2009;10: 52.). The transition to quantify the "classical" AD biomarkers amyloid and tau in blood has made advances in the last years (for a review see Chong et al., 2021). Nonetheless, the search for additional biomarkers continues.

Recent studies have shown that not only neurodegeneration but also immunological processes play a role in the development of AD. The deterioration of the immune system with age ("immunosenescence") leads to less effective removal of protein deposits. Inflammation increases and neurons die, as reported in various studies (Huang et al. Neurosci Lett. 2013 Aug 29;550:60-3.; Bagyinszky et al. J Neurol Sci. 2017 May 15;376:242-254; Su et al. Neurosci Bull. 2016 Oct;32(5):469-80).

There is a strong need to implement these aspects into AD diagnostics. First attempts have been made to find combinations of blood-based biomarkers that cover several aspects of AD (Schipke et al. Definition and quantification of six immune- and neuroregulatory serum proteins in healthy and demented elderly. Neurodegener. Dis. Manag. 2019, Aug., 9 (4): 193-203). This study includes a combination of six biomarkers: brain derived neurotropic factor (BDNF), insulin-like growth factor 1 (IGF-1), vascular endothelial growth factor (VEGF), transforming growth factor beta type 1 (TGF-beta1), monocyte chemoattractant protein 1 (MCP-1) and interleukin-18 (IL-18).

BDNF is a protein of the neurotrophin family of growths factors. It is involved in longterm memory processes, supporting cell and neuron survival. It thus has a neuroprotective function and helps to maintain neuronal function in the brain. BDNF has been found to modulate pro-inflammatory transcription factors such as NF-kB and AP-1 (Xu, D. et al. Brain-derived neurotrophic factor reduces inflammation and hippocampal apoptosis in experimental Streptococcus pneumoniae meningitis. J Neuroinflammation. 2017. 14(1):156). BDNF levels in CSF were shown to be decreased for AD patients. However, BDNF blood serum concentrations do not automatically correlate with BDNF levels in CSF (Laske et al. BDNF serum and CSF concentrations in Alzheimer's disease, normal pressure hydrocephalus and healthy controls. J Psychiatr Res. 2007 Aug;41(5):387-94).

IGF-1 is a hormone, induced by physical activity and low caloric intake. It is involved in neurogenesis, mediating neuroprotection and it supports cell survival. It is an antagonist to apoptosis. It has been found that lowered serum levels of IGF-1 correlate with an increased risk of developing AD dementia (Westwood et al. Neurology. 2014;82(18):1613-1619).

VEGF is an important signaling protein involved in the growth and maintenance of neuronal cells. Researchers found growing evidence, that VEGF plays a neuroprotective role, finding VEGF levels lowered in both serum and CSF among AD cases compared with controls (Huang et al. Neurosci Lett. 2013;550: 60-3.; Guo et al. Eur Arch Psychiatry Clin Neurosci. 2013; 263: 553-60.).

TGF-beta1 is a protein member of the cytokine family of transforming growth factors. TGF-beta1 controls cell growth, cell proliferation, differentiation and apoptosis, especially also for microglia cells. Lowered levels of TGF-beta1 have been shown to correlated with Amyloid beta accumulation and neurodegeneration. TGF-beta1 is regarded as one factor that controls the activity of microglia cells, which are responsible for phagocytosis of Amyloid-beta (Liu et al. Brain Res Bull. 2020 Mar; 156:86-104). There therefore seems to be evidence for TGF-beta1 to play a role in neuroprotection in AD, so that TGF-beta1 can be regarded as a biomarker for neuroinflammatory processes. Since there are already drugs that help increase TGF-beta1 levels in the central nervous system, the protein is a new target for further research related to AD and its treatment (Caraci et al. NS Neuroscience & Therapeutics, 2011 vol. 17, iss. 4, 237-246).

MCP-1 is a protein mediating the inflammatory response in AD. A recent study showed that blood plasma MCP-1 levels are higher in AD patients than in mild cognitive impairment (MCI) patients and controls. Moreover, it was found that the higher plasma MCP-1 levels were associated with faster cognitive decline. Thus, there seems to be a strong correlation between MCP-1 levels in blood plasma and the risk to develop AD or the aggravation of AD (Lee et al. Sci Rep 8, 1280 (2018)).

IL-18 cytokine plays an important role as regulators in inflammation and immune response. Recent findings indicate that IL-18 has an impact on the integrity of neurons and thus may contribute to the development of AD, too (Bossù et al. Curr Pharm Des. 2010;16(38):4213-24).

NfL has been investigated as a biomarker for neurodegenerative processes. It has emerged as a biomarker in cerebrospinal fluid (CSF) and plasma (Mattsson N, et al. JAMA Neurol. 2017;74: 557-566. and Kern Set al. JAMA Neurol. 2019;76: 187-193) and was shown to discriminate to some extent between AD and healthy controls (Lewczuk P, et al. Alzheimer's Res Ther. 2018;10: 71 and N Mattsson et al. JAMA Neurol. 2017;74: 557-566) in a preselected population of subjects. However, NfL has shown limited specificity to detect AD but rather neurodegenerative diseases in general and is especially increased in diseases such as amyotrophic lateral sclerosis or Creutzfeld-Jakob disease (Gaetani L, et al. J Neurol Neurosurg Psychiatry. 2019;90: 870-881). Increased values of NfL have previously been found in various neurological conditions, such as multiple sclerosis, motor neuron disease or frontotemporal dementia. CSF and serum NfL levels have been shown to be highly correlated (Halbgebauer S et al. Journal of Neurology, Neurosurgery & Psychiatry 2022;93:68-74). However, NfL shows only one aspect of AD. NfL was not known from the prior art to be useful for the diagnosis or risk assessment of AD in a population that was not specifically selected by AD stats, especially not for detection of early stages of the disease (see Sugarman et al. Neurobiol Aging. 2020 Oct; 94:60-70).). The analysis of only one biomarker does not seem to provide a safe strategy.

Studies examining novel blood-based biomarkers (BBM) (Popp J, et al. Brain Behav Immun. 2017;62: 203-211, O'Bryant SE, et al. J Alzheimer's Dis. 2014;42: 1325-1335 and O'Bryant et al. Alzheimer's Dement Diagnosis, Assess Dis Monit. 2016.) have found a promising diagnostic value of sets of serum protein biomarkers to distinguish between subjects impaired due to AD, both at dementia (Schipke CG, et al. Neurodegener Dis Manag. 2019;9: 193-203.) and mild cognitive impairment (MCI) (Schipke CG et al. Alzheimer Dis Assoc Disord. 2020;34: 318-324.) stage and healthy controls. One of these potential biomarkers is BDNF, which may reflect altered neurotrophic processes as a basis of AD-related cognitive impairment. BDNF is a protein involved in the maintenance of neuronal function and plastic changes related to learning and memory (Miranda M, et al. Front Cell Neurosci. 2019;13: 363). Lower BDNF concentrations in CSF (Laske C, et al. J Psychiatr Res. 2007;41: 387-394), serum (Ng TKS, et al. Int J Mol Sci. 2019;20: 257), and brain tissue (Connor B, et al. Mol Brain Res. 1997;49: 71-81) were described in AD. However, the level of BDNF might be dependent on the stage of AD progression, as there is evidence that in early stages it might be increased as a compensatory repair mechanism and only decrease in later stages (Laske C, et al. J Neural Transm. 2006;113: 1217-24). Further biomarkers of interest are TGF-β1, a cytokine involved in cell growth and apoptosis which is increased in AD patients' plasma (Malaguarnera L, et al. Neuropathology. 2006;26: 307-312), and IL-18, a proinflammatory cytokine increased in AD patients' serum (Chen JM, et al. Dement Geriatr Cogn Disord. 2014;38: 321-9.).

WO2015/113995 A1 describes a method for diagnosing or for determining the risk of developing AD in a subject comprising a) measuring the level of AD biomarker in a biological sample from a subject; and b) determining or diagnosing the presence or the risk of developing of AD with high specificity based on the level of said biomarker, whereby the AD biomarker are at least four biomarker selected from BDNF, IGF-1, TGF-beta 1, VEGF, IL-18, and MCP- 1, and whereby an increase of IL-18, and/or MCP-1 , and/or the decrease of BDNF, IGF-1 , VEGF and/or TGF-beta 1 is described to be indicative for the presence or risk of development of AD.

However, further optimization is strongly needed. The high specificity announced in WO2015/113995 A1 of at least 90% was reached on the basis of biological samples of specifically pre-selected patients. The patients recruited were aged between 57 and 95, wherein 10 out of 81 patients and thus a high percentage were older than 90 years old. Moreover, they had been selected due to a diagnosis of probable AD in a geriatric hospital unit. Finally, they passed further preselection steps according to the NINCDS-ADRDA Alzheimer Criteria (McKhann et al. Neurology. 1984 Jul;34(7):939-44). This means that an accuracy of more than 90% was reached when comparing a group of AD patients, who can be referred to as advanced in regard to their age and also in their stage of disease to a group of healthy individuals.

In some studies, patients are additionally recruited from long term AD studies (such as in Sugarman et al. Neurobiol Aging. 2020 Oct; 94:60-70). Thus, participants complete annual evaluations such as special interviews, neuropsychological and regular blood testing.

Patients are selected for a suspected etiology of AD. Patients with cognitive impairment due to non-AD conditions take part in the study, however only of a strongly reduced number.

This shows that the published data on biomarkers so far have mostly been derived from clinical study situations. Statistics thus reflect the clinical study situation. Statistical values, such as sensitivity (true positive rate) or specificity (true negative rate) thus always have to be regarded against the background of study participants. Special care therefore must be taken when evaluating or comparing results of clinical studies, in order not to compare data, which are based on completely different variables.

The data published so far, are based on clinical studies and based on patient cohorts as described above.

Under practical working conditions, for example for patients appointing for a special consultation in hospital or in case the general practitioner finds the patient's memory loss conditions suspicious, the biomarkers identified so far may be helpful for further diagnostics, but would not perform as described in specifically designed studies and would not provide sufficient sensitivity and/or specificity for the diagnosis and/or risk prediction of subjects outside of the selected patient populations described above.

Therefore, there is a strong need for further optimization. Blood-based tests must be improved in view of their accuracy especially for early stages of AD and before the onset of distinguishable clinical symptoms. They must prove to be workable, not only in highly specialized hospitals.

It is the aim of the invention to provide such a method.

### Summary of the invention

Subject matter of the invention is a method for
a) diagnosing Alzheimer's disease or
b) determining the risk of suffering from Alzheimer's disease
in a subject,
wherein the method comprises determining the level of at least the biomarkers Neurofilament light Chain (NfL), brain-derived neurotrophic factor (BDNF),
and tumor growth factor beta 1 (TGF-beta 1) and in addition determining the level of one or more biomarkers selected from the group comprising interleukin 18 (IL-18), Monocyte chemotactic protein-1 (MCP-1), Insulin-like growth factor (IGF) and Vascular endothelial growth factor (VEGF) in a sample of bodily fluid of said subject,
calculating a score from the determined biomarker levels, and
comparing said score with a reference score,
and wherein said subject is diagnosed with Alzheimer's disease, or said subject is determined as having a risk of suffering from Alzheimer's disease, if the score is above said reference score.

Particular embodiments of the invention relate to aspect a), diagnosing Alzheimer's disease.

In certain aspects, the invention relates to a method comprising preparing a sample wherein said sample comprises bodily fluid from a subject, and binders binding to the biomarkers Neurofilament light Chain (NfL), brain-derived neurotrophic factor (BDNF), and tumor growth factor beta 1 (TGF-beta 1) respectively, and wherein said sample additionally comprises binders binding to one or more secondary biomarkers selected from the group consisting of interleukin 18 (IL-18), Monocyte chemotactic protein-1 (MCP-1), Insulin-like growth factor (IGF), and Vascular endothelial growth factor (VEGF) respectively,
wherein said subject has Alzheimer's disease or is at risk of suffering from Alzheimer's disease if a score calculated from the levels of said biomarkers and said one or more secondary biomarkers in said sample is above a predetermined reference score or wherein said subject does not have Alzheimer's disease or is not at risk of suffering from Alzheimer's disease if a score calculated from the levels of said biomarkers and said one or more secondary biomarkers in said sample is below a predetermined reference score; wherein said predetermined reference score is obtained by training a machine-learning algorithm with output data for the levels of said biomarkers and said one or more secondary biomarkers obtained from a reference group of subjects, and wherein said score is calculated from the levels of said biomarkers and said one or more secondary biomarkers by factoring in the level of each said biomarker and one or more secondary biomarkers in said sample with a respective individual factor obtained from the output generated by said machine learning algorithm.

In certain embodiments, said method comprises receiving, in a processing device, measurement data indicative of the determined levels of said biomarkers and said one or more secondary biomarkers in said sample,
calculating, as detailed herein, said score in the processing device,
comparing said score to said reference score stored in the processing device,
generating output data relating to said subject having Alzheimer's disease or being at risk of suffering from Alzheimer's disease based on said score being above said reference score, or generating output data relating to said subject not having Alzheimer's disease or not being at risk of suffering from Alzheimer's disease based on said score being below said reference score,
and outputting the output data via an output device, such as a display or printer.

Further specific embodiments of said method are defined by the subject matter as detailed further herein below.

In certain embodiments of the invention, the predetermined reference score is at the 50^{th} percentile of the scores determined in the training subjects as detailed herein, which can e.g. be expressed as a score of 0.5.

In certain embodiments of the invention, the method may involve two predetermined reference scores having different values, i.e. an upper predetermined reference score and a lower predetermined reference score, and wherein said attribution of said subject having Alzheimer's disease or being at risk of suffering from Alzheimer's disease is if the score for said subject is above the upper predetermined reference score or wherein said attribution of said subject not having Alzheimer's disease or not being at risk of suffering from Alzheimer's disease is if the score for said subject is below the lower predetermined reference score. In particular embodiments the upper reference score is at the 80^{th} percentile of the scores determined in the training subjects as detailed herein, which can e.g. be expressed as a score of 0.8, and the lower reference score is at the 30^{th} percentile of the scores determined in the training subjects as detailed herein, which can e.g. be expressed as a score of 0.3.

In certain embodiments, the score is assigned to the sample, based on the calculation as described herein. In particular embodiments, said score assigned to said sample is equal or above the 50^{th} percentile, as described further herein, and said subject has AD, or is classified as having AD.

In certain embodiments, the score is assigned to the sample, based on the calculation as described herein. In particular embodiments, said score assigned to said sample is equal or above the 80^{th} percentile, as described further herein, and said subject has AD, or is classified as having AD.

In certain aspects, the invention relates to a method of treatment of a subject diagnosed with Alzheimer's disease, or determined as having a risk of suffering from Alzheimer's disease, wherein said diagnosis or determination is made with the method of diagnosing Alzheimer's disease or determining the risk of suffering from Alzheimer's disease according to the present invention as detailed herein, and wherein a (prophylactic) treatment against Alzheimer's disease is administered to said subject. Such Alzheimer's treatments are known to the skilled person.

As used herein "said subject is diagnosed with Alzheimer's disease" may in certain embodiments also be expressed as "said subject is characterized as having Alzheimer's disease". Typically, the subjects in the present invention are human subjects.

In certain embodiments of the invention, the score is calculated with an algorithm. In certain embodiments of the invention, the algorithm is calculated from the data obtained from a reference group of subjects, in particular by a statistical method, more particularly a prediction computational model based on machine learning. For sake of distinction, subjects in the reference group are also referred to herein as "training subjects".

In certain embodiments of the present invention, the biomarkers as detailed herein are examined in unstratified patients with a wide variety of diagnoses and healthy controls, in particular in the reference group. This is as opposed to cohorts of related research questions (see e.g. Jessen F, et al. Alzheimer's Res Ther. 2018;10: 15., Weiner MW, et al. Alzheimer's Dement. 2015;11: e1-e120. and Rowe CCet al. Neurobiol Aging. 2010;31: 1275-1283.).

In certain embodiments of the invention, the reference group comprises cognitively healthy training subjects and cognitively impaired training subjects (e.g. as assessed by a physician by art-known methods, such as the Mini-Mental Status Examination (MMSE), Montreal Cognitive Assessment (MoCA), as art-known questionnaire on cognitive decline and/or diagnosed according to the fifth edition of the Diagnostic and Statistical Manual of Mental Disorders (DSM-5)). The ratio of cognitively healthy training subjects and cognitively impaired training subjects may be chosen as is suitable, and may be e.g. about 1:3, about 2:3, about 1:2, about 1:1, about 2:1, about 3:2, or about 3:1, preferably about 1:1 (each specifying the ratio of healthy to cognitively impaired training subjects).

In certain embodiments of the invention, the reference group may exclude certain training subjects, e.g. based on certain traits such as gender, ethnicity, age group, BMI, and known risk factors for AD. For instance, the reference group may be a group of training subjects having one or more traits, such as the aforementioned, in common with the subject that is to be analyzed with the method of the invention, e.g. within the same age group, e.g. within the same 5-year group (60-65, 65 to 70 and so on). Also, subjects with pre-existing conditions such as renal insufficiency, HIV or other autoimmune diseases, acute infections, acute stroke or stroke with residual symptoms, substance abuse or psychotic disorder typically may be excluded in certain embodiments. Furthermore, patients with regular statin intake may be excluded as it was shown that statins may influence BDNF, VEGF or IGF-1 values (Höglund et al. Exp Brain Res 164, 205-214 (2005); Bergen et al. Growth Horm IGF Res. 2016 Aug. 29:78-82; Sahebkar et al. 2015 Nov;64(11):1466-76).

The levels of the biomarkers as detailed in the embodiments of the invention are determined for each training subject of the reference group to obtain a training data set. A statistical method is then applied to the training data set obtain an algorithm for calculating the score and to obtain a reference score. In certain embodiments of the invention, the statistical method may be a prediction computational model based on machine learning including at least one classification method, and/or may be selected from the group comprising multiple logistic regression, principle component analysis, K-nearest neighbor algorithm, unsupervised clustering, deep neural networks , elastic net, decision trees algorithm, random forest algorithm, and support vector machines, preferably multiple logistic regression, elastic net, decision trees algorithm, random forest algorithm, and support vector machines, more preferably multiple logistic regression.

The statistical method is used to predict a single binary variable (i.e. has AD / not have AD; or has a risk / does not have a risk for suffering from AD).

In certain embodiments, said algorithm is optimized to increase the sensitivity (true positive rate) of the method of the invention. In certain embodiments, said algorithm is optimized to increase the specificity (true negative rate) of the method of the invention. In further embodiments, said algorithm is optimized to increase the sensitivity (true positive rate) and the specificity (true negative rate) of the method of the invention.

In certain embodiments, the reference score is set at a value that optimizes the specificity (true negative rate) of the method of the invention.

In certain embodiments, the reference score is set at a value that optimizes the sensitivity (true positive rate) of the method of the invention. In further embodiments, the reference score is set at a value that optimizes the sensitivity (true positive rate) and the specificity (true negative rate) of the method of the invention.

As used herein, "score" and "reference score", respectively, are in particular numerical values calculated from the levels of the biomarkers as detailed in the embodiments of the invention and as obtained from the (training) subjects (e.g. by applying an algorithm as detailed herein). Typically, such (reference) scores are dimensionless, meaning they have no unit (e.g. relating to a concentration, amount or the like) attached. The (reference) scores may in relation to a normalized scale, meaning that the lowest score obtainable from the biomarker data is set to a fixed value, e.g. 0, and the highest score obtainable from the biomarker data is set to a second fixed value, e.g. 1 or 100. Typically, the highest and lowest score obtainable from the biomarker data, and thus the normalization, would be based on the scores obtained from a reference group of training subjects, as detailed herein. The reference score can then be expressed as a value within this (normalized) scale, which could be a plain numerical value, or a percentage or percentile value.

In certain embodiments of the invention, two reference scores are employed in the method, which are determined as described herein; a first reference score is optimized for sensitivity, whereas the second reference score is optimized for specificity; the score is compared with both the first and second reference score, and said subject is diagnosed with Alzheimer's disease, or said subject is determined as having a risk of suffering from Alzheimer's disease, if the score is above said first reference score, and said subject is diagnosed with not having Alzheimer's disease, or said subject is determined as not having a risk of suffering from Alzheimer's disease, if the score is below said second reference score. In certain embodiments of the invention, if said score is in the range from the second to the first reference score (i.e. between and including the respective reference scores), no diagnosis or determination of risk is made for said subject and said subject is scheduled for retesting with the method of the invention, as described herein.

In certain embodiments of the invention, if the subject's score is at or above a certain percentile, e.g. the 50^{th}, the 60^{th}, the 65^{th}, the 70^{th}, the 75^{th}, the 80^{th}, the 85^{th}, the 90^{th}, or the 95^{th} percentile, particularly the 70^{th}, the 75^{th}, the 80^{th}, the 85^{th}, the 90^{th}, or the 95^{th} percentile, more particularly the 75^{th}, the 80^{th}, or the 85^{th}, percentile, said subject is diagnosed with Alzheimer's disease, or said subject is determined as having a risk of suffering from Alzheimer's disease.

In certain embodiments of the invention, if the subject's score is at or below a certain percentile, e.g. the 50^{th}, the 45^{th}, the 40^{th}, the 35^{th}, the 30^{th}, the 25^{th}, the 20^{th}, the 15^{th}, or the 10^{th} percentile, particularly the 20^{th}, the 15^{th}, or the 10^{th} percentile, more particularly the 30^{th}, the 25^{th}, or the 20^{th} percentile said subject is diagnosed as not having Alzheimer's disease, or said subject is determined as not having a risk of suffering from Alzheimer's disease.

In certain embodiments of the invention, if the subject's score is withing a certain range (an "intermediate range"), e.g. from the 20^{th} to 70^{th}, from the 25^{th} to 65^{th}, from the 30^{th} to 60^{th}, from the 35^{th} to 55^{th}, from the 40^{th} to 50^{th} percentile, particularly from the 20^{th} to 70^{th}, from the 25^{th} to 65^{th}, or from the 30^{th} to 60^{th} percentile, more particularly from the 25^{th} to 80^{th}, from the 30^{th} to 75^{th}, or from the 35^{th} to 65^{th} percentile, no diagnosis or determination of risk is made for said subject and said subject is scheduled for retesting with the method of the invention, as described herein.

The aforementioned percentile values are in relation to the scores that can be calculated for the training subjects as described herein. As an example, if the scores are determined for a group of training subjects, and the scores obtained for the training subjects are normalized on a scale of 0 to 1 (0 being assigned to the subject with the lowest score and 1 being assigned to the subject with the highest score), the nth percentile would correspond to a score of 0.n (e.g. 80^{th} percentile = 0.80).

In certain embodiments of the invention, the method according to the invention, including the determination of the levels the biomarkers according to the method as detailed herein in a sample of bodily fluid of said subject, calculating a score from the determined biomarker levels, and comparing said score with a reference score is performed more than once in said subject, e.g. in an interval of one month, two months, five months, 8 months, 12 months, 18 months, 2 years, 3, 4, 5 or 6 years, particularly 18 months. This may allow for a monitoring of a change and/or the progression of the of the subject's AD status. In particular embodiments, this may be performed in subjects where the first execution of the method of the invention on said subject yields a score that does not allow for diagnosing or diagnosing with sufficient certainty said subject with Alzheimer's disease (or with not having AD), or determining or determining with sufficient certainty said subject as having a risk of suffering from AD (or as not having a risk of suffering from AD), respectively.

In certain embodiments of the invention, the score is calculated by means of a computational step, which is implemented in a processing device.

In certain embodiments of the invention, the method is a computer implemented method comprising receiving, in a processing device, data (e.g. as numerical values) of the determined levels of each of the biomarkers,
calculating, in the processing device, a score from the determined biomarker levels, and
comparing, in the processing device, said score with a reference score (which may e.g. be obtained from a data base or obtained as a measured level from a control, preferably from a data base), analyzing whether said score is above or below the reference score, and assigning said subject as being diagnosed with Alzheimer's disease, or assigning said subject as having a risk of suffering from Alzheimer's disease, if said score is above said reference score.

The computer implemented method may furthermore comprise outputting data on an output unit, such as a screen or a printer. The data output may comprise the score, the relation to the reference score and/or the result of whether the subject is the assignment as being diagnosed with Alzheimer's disease, the determination as (not) having a risk of suffering from Alzheimer's disease; a readable result will be presented, for example, the readable result will be provided as a graphical display, preferably as a colored bar, indicating to the user whether the dataset of the biological sample indicates (i) AD or (ii) non-AD. An area may additionally be presented in between of the two classes (i) AD and (ii) non-AD in order to give a further indication to the user that the result obtained needs further examination. In this case, for example, a recommendation to complete further diagnostic steps will be given to the user.

The processing device may comprise or be a personal computer, mobile device such as mobile phone or smartphone, personal digital assistant (PDA), tablet computer, notebook, or any other device suitable electronically performing the steps according to the invention as described above.

In further embodiments, the computer implemented method according to the invention may be provided, the method comprising that in step c) the reference score is obtained from a control population.

In other embodiments of the invention, a computer implemented method may be provided, the method comprising:
- receiving, in a processing device, measurement data indicative of a measurement of the level of at least three biomarkers NfL, BDNF, and TGF-beta 1 and in addition one or more biomarkers selected from the group comprising IL-18, MCP-1, IGF and VEGF;
- predicting whether a subject is diagnosed with Alzheimer's disease or a subject is at risk of suffering from Alzheimer's disease using a machine-learning algorithm trained with training data comprising output synthetic data from a reference group of subjects;
- generating output prediction data indicative of the predicted value for determining whether the subject is diagnosed with Alzheimer's disease or whether the subject is at risk of suffering from Alzheimer's disease; and
- outputting the output prediction data via a processing device.

In embodiments, a computer program product comprising instructions which, when the program is executed by a computer, cause the computer to carry out the steps of the method according to the invention may be provided.

In other embodiments, a computer readable medium or computer program product having computer-executable instructions for performing the steps as identified in the invention may be provided.

In certain embodiments of the invention, the method comprises determining the level of at least the biomarkers NfL, BDNF, TGF-beta 1 and IL-18.

In certain embodiments of the invention, the method comprises determining the level of at least the biomarkers NfL, BDNF, TGF-beta 1 and MCP-1.

In certain embodiments of the invention, the method comprises determining the level of at least the biomarkers NfL, BDNF, TGF-beta 1 and IGF.

In certain embodiments of the invention, the method comprises determining the level of at least the biomarkers NfL, BDNF, TGF-beta 1 and VEGF.

In preferred embodiments of the invention, the method comprises determining the level of at least the biomarkers NfL, BDNF, TGF-beta 1 and IL-18, or NfL, BDNF, TGF-beta 1 and MCP-1, more particularly NfL, BDNF, TGF-beta 1 and IL-18.

In an embodiment of the invention, when calculating the score, the level of NfL is increased and, at the same time, the levels of BDNF and TGF-beta1 are decreased indicating that the subject is diagnosed with AD.

As known to the skilled person, age is an important risk factor for Alzheimer disease.

The risk doubles every 5 years from an age of 65 on (Patterson et al. Age and amyloid effects on human central nervous system amyloid-beta kinetics. Ann Neurol. 2015;78(3):439-453). In certain embodiments of the invention, the method comprises adjusting the score for age. In certain embodiments, the reference score is calculated based on a reference group of the same age group (e.g. within the same 5-year group, e.g. 60-65, 65 to 70 and so on) as the subjects to be tested with the method of the invention. In certain embodiments of the invention, no age-adjustment is applied for the biomarkers BDNF, TGF-beta1, IGF-1, MCP-1, and/or VEGF. Unexpectedly, these biomarkers have been shown to be stable and the method of the invention, with respect to the quantification of these biomarkers, thus is independent of age-related impacts. The data collected therefore can be processed independently from the age of the patient and there are no age-related changes that have to be taken into account during calculations.

In certain embodiments of the invention, the calculation of the score and/or the reference score includes weighting factors for one or more of the determined biomarkers, such as for the subject's age, race, Body mass index (BMI), mental status testing (e.g. mini-mental state examination (MMSE)), neuroimaging (CT, MRT, PET, SPECT), family history, ApoE4 genotype, Amyloidβ 1-42 (Aβ₁₋₄₂), Amyloidβ 1-40 (Aβ₁₋₄₀), total Tau-protein, phosphorylated Tau-protein (p-Tau 181, p-Tau 199, p-Tau 231), co-morbidities such as vascular diseases, history of stroke, psychiatric diseases, or intake of medication such as statins, acetylcholine esterase inhibitors or any medication intended to lowering blood pressure.

Another finding was, that serum NfL levels change (increase) with aging (Khalil, M. et al. Nat Commun 11, 812 (2020)). It was found by the present inventors upon further studies that NfL increases by a median of 3.27% with each year of age. In certain embodiments, determined NfL levels are adjusted with a correction factor to compensate for the subject's age, in particular to compensate for an increase of about 3, particularly about 3.2 or about 3.3, more particularly 3.27% per year of the subject's age.

Serum IL-18 levels may also change (increase) with aging (Menne, F. et al. Biomark Med. 2022 May;16(7):511-521), increasing about 3% each year. In certain embodiments, determined IL-18 levels are adjusted with a correction factor to compensate for the subject's age, in particular to compensate for an increase of about 3% per year of the subject's age.

In certain embodiments of the invention, the subject that has previously not been (i.e. has never been) diagnosed with Alzheimer's disease at the time of sample taking. In certain embodiments of the invention, the subject that has previously not been diagnosed with MCI. In certain embodiments of the invention, the subject that has previously not been diagnosed with any form of cognitive impairment at the time of sample taking.

In certain embodiments of the invention, the subject has mild cognitive impairment (MCI). In certain embodiments of the invention, the subject shows signs of cognitive impairment. MCI and/or dementia are particularly determined with a standardized neuropsychometric test, e.g. as described herein. The test may be selected from the group comprising Mini-Mental State Examination (MMSE); a test assessing depressive symptoms (e.g. Geriatric Depression Scale [GDS]); any test assessing the activities of daily living (e.g. an interview for deterioration in daily living activities [IDDD]); a test assessing verbal learning and episodic memory (e.g. evaluated with the Consortium to establish a registry for Alzheimer's disease [CERAD] word list and Wechsler Memory Scale III logical memory subtest); a test assessing attention and executive function (e.g. digits test from Wechsler adult intelligence scale III [WAIS-III], trail making test B [TMT-B], phonetic fluency (p), Stroop test, constructional praxis from CERAD); a test assessing visual perception (e.g. letters test from the visual object and space perception battery [VOSP]); a test assessing visuo-spatial function (e.g. number localization task from VOSP); a test assessing agnosia (e.g. Poppelreuter figures test; psychomotor speed (TMT-A); or a test assessing semantic fluency (e.g. animal categories) and language (e.g. Boston naming test [BNT]).

In certain embodiments of the invention, the method is used to stratify the subjects into risk groups, such as subjects with a low risk, medium risk, or high risk to suffer from Alzheimer's disease. In certain embodiments, low risk of suffering from Alzheimer's disease in particular means that the score determined by the method according to the invention, is at the reference score or above and within a margin of 10, 9, 8, 7, 6, 5, 4, 3, 2 or 1 percentage points of the reference score.

A medium risk means the score is above the range for a low risk and within a margin of 20, 19, 18, 17, 16, 15, 14, 13, 12, 11 percentage points of the reference score. A high risk means the score is above the range for a medium risk.

As used herein, "significantly increased" in particular indicates a p value of ≤ 0.05.

In certain embodiments of the invention, risk of suffering from Alzheimer's disease means the risk of suffering from Alzheimer's disease within a certain period of time.

The term "risk", as used herein, relates to the probability of suffering from Alzheimer's disease.

In certain embodiments of the invention, the reference score is a predetermined reference score.

In certain embodiments of the invention, the levels of the biomarkers of the invention are determined with an assay such as an immunoassay, e.g. as described in the examples. In other embodiments, the biomarker levels are determined by mass spectrometry, or alternatively any other suitable method.

In certain embodiments of the invention, calculation of the score may comprise a correction factor. The correction factor may take into account the specific method used for determining the biomarker levels; a predetermined correction factor may be applied, which is e.g. based on a calibration, comparing scores obtained with different methods used for determining the biomarker levels, or any other factors that may have an impact on determining the biomarker levels (devices, materials, reagents, buffers, etc.).

The person skilled in the art knows how to determine reference scores from previously conducted studies. The person skilled in the art knows that a specific reference score may depend on the cohort used for calculating a reference score that can be later-on used in the method of the invention. The person skilled in the art knows that a specific reference score may depend on the calibration used in the assay.

The person skilled in the art knows that a specific reference score may depend on the sensitivity and/or specificity that seems to be acceptable for the medical practitioner. The sensitivity and specificity of a diagnostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy) and "disease" populations (i.e. patients suffering from Alzheimer's disease). Depending on the particular diagnostic question to be addressed, the reference group must not be necessarily "normal", but it might be a group of patients suffering from another disease or condition, from which the diseased group of interest shall be differentiated. For any particular marker, a distribution of biomarker levels for subjects with and without a disease will likely overlap. Under such conditions, a test does not absolutely distinguish normal from disease with 100% accuracy, and the area of overlap indicates where the test cannot distinguish normal from disease. A reference score is selected, above which (or below which, depending on how a biomarker changes with the disease) the test is considered to be abnormal and below which the test is considered to be normal. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art (See, e.g., Hartley et al, 1982). Preferably, a reference score is selected to provide a ROC curve area of greater than about 0.75, more preferably greater than about 0.80. The term "about" in this context refers to +/- 5% of a given measurement.

As used herein, "specificity" of a method of the invention refers to the method's true negative rate, i.e. the probability of a negative test result, conditioned on the tested subject truly being negative.

In certain embodiments of the invention, the bodily fluid is selected from the group comprising whole blood, serum, plasma, nasal secretion, cerebrospinal fluid (CSF) and saliva, particularly whole blood, serum, plasma, and cerebrospinal fluid (CSF), more particularly whole blood, serum, and plasma, even more particularly serum.

Measurements of NfL, BDNF, TFG-beta, MCP-1 and IL-18 in saliva were reported in Gleerup, Helena Sophia et al. Frontiers in aging neuroscience vol. 13 659898. 10 May. 2021, La Fratta, I et al. Scientific reports vol. 8,1 3031. 14 Feb. 2018 and Ruacho, Guillermo et al. Lupus science & medicine vol. 9,1 (2022): e000607. Measurements of NfL, BDNF, TFG-beta, MCP-1 and IL-18 in urine were reported in Ruacho, Guillermo et al. Lupus science & medicine vol. 9,1 (2022): e000607, Sirota, Jeffrey C et al. BMC nephrology vol. 14 17. 17 Jan. 2013, Siriwattanasit, Narongrit et al. BMC nephrology vol. 22,1 236. 26 Jun. 2021, Covarrubias, Claudia et al. Metabolites vol. 13,6 723. 3 Jun. 2023 and Kohlhase K, et al. Eur J Neurol. 2023 Mar;30(3):729-740. Measurements of NfL, BDNF, TFG-beta, MCP-1 and IL-18 in CSF were reported in Rauch, Jessica et al. Emerging microbes & infections vol. 11,1 (2022): 1843-1856 and Gaetani L, et al. J Neurol Neurosurg Psychiatry. 2019 Aug;90(8):870-881. doi: 10.1136/jnnp-2018-320106. Epub 2019 Apr 9. Measurements of NfL, BDNF, TFG-beta, MCP-1 and IL-18 in nasal secretion were reported in Krakowiak A, et al. Int J Occup Med Environ Health. 2008;21(2): 165-72, Fang, Li et al. Journal of inflammation research vol. 16 2595-2606. 19 Jun. 2023, Ciprandi G, et al. Pediatr Allergy Immunol. 2004 Apr;15(2):148-51 and Li Y, et al. Inflammation. 2016 Aug;39(4):1582-93.

A variety of immunoassays are known and may be used for the methods of the invention, these include in particular: radioimmunoassays ("RIA"), homogeneous enzyme-multiplied immunoassays ("EMIT"), enzyme linked immunoadsorbent assays ("ELISA"), apoenzyme reactivation immunoassay ("ARIS"), dipstick immunoassays and immuno-chromotography assays.

According to a preferred embodiment of the invention, the immunoassay is performed as a multiplex assay or as a singleplex assay. A multiplex immunoassay allows the quantification of multiple biomarkers in a single assay. Usually, magnetic beads or other solid surfaces are used to immobilize binders for the biomarkers to be analyzed. Even more preferred, according to the invention, is the use of a Simple Plex assay. Preferable, this assay is conducted on an array, providing single multifluidic channels for each biomarker to be assessed. For example, an immunoassay according to the Ella^{®} System (ProteinSimple, Biotechne) is employed. Advantageously, no cross-reactions between different antibodies are present. In such assays, only very small sample volumes are required for generating highly accurate results for quantification.

Since results of biomarker determination may differ between different assay formats used (see e.g. Menne F, et al. Bioanalysis 2023 Oct;15(19):1157-1167), in particular embodiments the assay used for determining the score and the reference score is the same. In other particular embodiments one or more correction factors are applied to compensate for the deviations between assays; the skilled person can determine such correction factors by a routine comparative analysis of the assays used, which may e.g. involve standardized samples of the biomarkers.

It is an object of the invention to provide respective kits that can be used in the methods of the invention. Subject matter of the invention is a kit comprising one or more binders that bind specifically to NfL, one or more binders that bind specifically to BDNF, one or more binders that bind specifically to TGF-beta 1,
and one or more binders that bind specifically to a biomarker selected from the group comprising IL-18, MCP-1, IGF and VEGF, particularly IL-18, and optionally comprising instructions on how to use the kit in a method of the invention.

In the context of the invention, "binders" or "binder molecules" are molecules which may be used to bind target molecules or molecules of interest, i.e. analytes (i.e. in the context of the invention in particular molecules of interest are NfL, BDNF, TGF beta 1, IL-18, MCP-1, IGF and VEGF, more particular NfL, BDNF, TGF beta 1, IL-18, and MCP-1, even more particular NfL, BDNF, TGF beta 1 and IL-18, in a sample. Binder molecules have thus to be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the invention, binder molecules may for instance be selected from the group comprising a nucleic acid molecule, a carbohydrate molecule, a PNA molecule, a protein, an antibody, a peptide or a glycoprotein.

Preferably, the binder molecules are antibodies, including fragments thereof with sufficient affinity to a target or molecule of interest, and including recombinant antibodies or recombinant antibody fragments, as well as chemically and/or biochemically modified derivatives of said antibodies.

The kit may additionally comprise a calibrator. Samples with known concentrations of NfL, BDNF, TGF-beta 1, IGF, VEGF, MCP-1 and/or IL-18 (native, purified or recombinant) may serve as calibrators.

The kit may also comprise one or more washing reagents such as aqueous buffer solutions with or without detergent. Examples comprise aqueous buffer solutions with e.g. Tris-HCl, NaCl and Tween 20.

Subject matter of invention is a kit, wherein the binders are selected from the group comprising antibodies, antibody fragments and non-Ig scaffolds.

In a specific embodiment of said kit said binders are antibodies.

The term "antibody" generally comprises monoclonal and polyclonal antibodies and binding fragments thereof, in particular Fc-fragments as well as so called "single-chain-antibodies" (Bird et al. 1988), chimeric, humanized, in particular CDR-grafted antibodies, and dia or tetrabodies (Holliger et al. 1993). Also comprised are immunoglobulin-like proteins that are selected through techniques including, for example, phage display to specifically bind to the molecule of interest contained in a sample. In this context the term "specific binding" refers to antibodies raised against the molecule of interest or a fragment thereof. An antibody is considered to be specific, if its affinity towards the molecule of interest or the aforementioned fragment thereof is at least preferably 50-fold higher, more preferably 100-fold higher, most preferably at least 1000-fold higher than towards other molecules comprised in a sample containing the molecule of interest. It is well known in the art how to make antibodies and to select antibodies with a given specificity. Commercially available antibodies are depicted below in table 1.

An antibody according to the invention is a protein including one or more polypeptides substantially encoded by immunoglobulin genes that specifically bind an antigen. The recognized immunoglobulin genes include the kappa, lambda, alpha (IgA), gamma (IgG1, IgG2, IgG3, IgG4), delta (IgD), epsilon (IgE) and mu (IgM) constant region genes, as well as the myriad immunoglobulin variable region genes. Full-length immunoglobulin light chains are generally about 25 kD or 214 amino acids in length. Full-length immunoglobulin heavy chains are generally about 50 kD or 446 amino acid in length. Light chains are encoded by a variable region gene at the NH2-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')2, as well as bifunctional hybrid antibodies and single chains (e.g., Lanzavecchia et al. 1987; Huston et al. 1988; Bird et al. 1988; Hood et al.1984; Hunkapiller & Hood, 1986). An immunoglobulin light or heavy chain variable region includes a framework region interrupted by three hypervariable regions, also called complementarity determining regions (CDR's) (see, Kabat et al. 1983).

As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art, e.g., see U.S. Patent No. 5,807,715. A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, i.e., at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering (e.g., see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest.

Immortalization can be accomplished, for example, by EBV infection or by fusing a human B cell with a myeloma or hybridoma cell to produce a trioma cell. Human antibodies can also be produced by phage display methods (see, e.g., PCT Publication No. WO91/17271; PCT Publication No. WO92/001047; PCT Publication No. WO92/20791, which are herein incorporated by reference), or selected from a human combinatorial monoclonal antibody library (see the Morphosys website). Human antibodies can also be prepared by using transgenic animals carrying a human immunoglobulin gene (for example, see PCT Publication No. WO93/12227; PCT Publication No. WO91/10741, which are herein incorporated by reference).

Thus, the binder used in the kit according to the invention may have the formats known in the art. Examples are human antibodies, monoclonal antibodies, humanized antibodies, chimeric antibodies, CDR-grafted antibodies. In a preferred embodiment antibodies according to the invention are recombinantly produced antibodies as e.g. IgG, a typical full-length immunoglobulin, or antibody fragments containing at least the F-variable domain of heavy and/or light chain as e.g. chemically coupled antibodies (fragment antigen binding) including but not limited to Fab-fragments including Fab minibodies, single chain Fab antibody, monovalent Fab antibody with epitope tags, e.g. Fab-V5Sx2; bivalent Fab (mini-antibody) dimerized with the CH3 domain; bivalent Fab or multivalent Fab, e.g. formed via multimerization with the aid of a heterologous domain, e.g. via dimerization of dHLX domains,e.g. Fab-dHLX-FSx2; F(ab')2-fragments, scFv-fragments, multimerized multivalent or/and multispecific scFv-fragments, bivalent and/or bispecific diabodies, BITE^{®} (bispecific T-cell engager), trifunctional antibodies, polyvalent antibodies, e.g. from a different class than G; single-domain antibodies, e.g. nanobodies derived from camelid or fish immunoglobulins and numerous others.

In addition to binder used in the kit according to the invention other biopolymer scaffolds are well known in the art to complex a target molecule and have been used for the generation of highly target specific biopolymers. Examples are aptamers, spiegelmers, anticalins and conotoxins.

Non-Ig scaffolds may be protein scaffolds and may be used as antibody mimics as they are capable to bind to ligands or antigens. Non-Ig scaffolds may be selected from the group comprising tetranectin-based non-Ig scaffolds (e.g. described in US 2010/0028995), fibronectin scaffolds (e.g. described in EP 1266 025; lipocalin-based scaffolds ((e.g. described in WO 2011/154420); ubiquitin scaffolds (e.g. described in WO 2011/073214), transferring scaffolds (e.g. described in US 2004/0023334), protein A scaffolds (e.g. described in EP 2231860), ankyrin repeat based scaffolds (e.g. described in WO 2010/060748), microprotein (preferably microproteins forming a cystine knot) scaffolds (e.g. described in EP 2314308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1941867). Non-Ig scaffolds may be peptide or oligonucleotide aptamers. Aptamers are usually created by selecting them from a large random sequence pool and are either short strands of oligonucleotides (DNA, RNA or XNA; Xu et al. 2010, Deng et al. 2014) or short variable peptide domains attached to a protein scaffold (Li et al. 2011).

In one embodiment of the invention binder used in the kit according to the invention according to the invention may be produced recombinantly. Suitable antibodies for the methods of the invention (which are then preferably produced recombinantly) may be determined by a method of fusing splenocytes from a mammal, preferably a rodent, previously immunized with a molecule of interest and myeloma cells, and selecting hybrid clones expressing antibodies against the molecule of interest as follows:
Mice are immunized with a molecule of interest or a fragment thereof, which may be conjugated to a serum protein, e.g. BSA, e.g. at day 0 at day 14 and at day 21 and 28. At day 49 the animal may receive an intravenous (i.v.) injection of of the molecule of interest or a fragment thereof, which may be conjugated to a serum protein, e.g. BSA, e.g. dissolved in saline. Subsequently, e.g. three days later the mice are sacrificed and splenocytes from the immunized mice and cells of a myeloma cell line, e.g. SP2/0, are fused (e.g. with 50% polyethylene glycol at 37°C). After washing, the cells are seeded (e.g. in 96-well cell culture plates). Hybrid clones are selected (e.g. by growing in HAT medium [RPMI 1640 culture medium supplemented with 20% fetal calf serum and HAT-Supplement]). Subsequently, e.g. after one week, the medium may be replaced (e.g. with HT Medium) for several, e.g. three passages, followed by returning to the normal cell culture medium. The cell culture supernatants are then primarily screened for recombinant IgG antibodies binding the molecule of interest, e.g. two weeks after fusion. Therefore, recombinant molecule of interest, tagged e.g. with GST, is immobilized, e.g. in 96-well plates, and incubated with cell culture supernatant per well, e.g. for 2 hours at room temperature. After washing of the plate, a detection antibody,
e.g. POD-rabbit anti mouse IgG, is added and incubated, e.g. for 1 h at RT. After a next washing step, the detection antibody is determined with known methods, e.g. via adding a chromogen solution (o-phenylendiamin in citrate/ hydrogen phosphate buffer) , incubating, e.g. for 15 minutes at RT, and stopping the chromogenic reaction, e.g. by the addition of 4N sulfuric acid, and detecting absorption at 490 mm. The positive tested microcultures are then propagated.

After retesting the selected cultures are cloned and recloned, e.g. using the limiting-dilution technique; isotypes may be determined. The skilled person is aware of how to modify such methods with known procedures and/or reagents and/or components.

Suitable antibodies (which are then preferably produced recombinantly) may alternatively be determined by means of phage display, e.g. according to the following procedure:
One or more human naive antibody gene library, e.g. HAL7/8, are used for the isolation of recombinant single chain F-Variable domains (scFv) against the molecule of interest or a fragment thereof. The antibody gene libraries are screened with a panning strategy comprising the use of peptides containing a biotin tag linked via two different spacers to the molecule of interest. A mix of panning rounds using non-specifically bound antigen and streptavidin bound antigen are used to minimize background of non-specific binders. The eluted phages from the third round of panning are used for the generation of monoclonal scFv expressing cells, e.g. E.coli strains. Supernatant from the cultivation of these clonal strains can be directly used for an antigen ELISA testing (see also Hust et al. 2011; Schütte et al. 2009)

Humanization of murine antibodies may be conducted according to the following procedure: For humanization of an antibody of murine origin the antibody sequence is analyzed for the structural interaction of framework regions (FR) with the complementary determining regions (CDR) and the antigen. Based on structural modeling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modeling. (Almagro & Fransson 2008).

In an alternative embodiment the binder used in the kit according to the invention is selected from the group comprising Fv fragment, scFv fragment, Fab fragment, scFab fragment, F(ab)2 fragment and scFv-Fc Fusion protein.

In another preferred embodiment the antibody format is selected from the group comprising scFab fragment, Fab fragment, scFv fragment and bioavailability optimized conjugates thereof, such as PEGylated fragments.

In certain embodiments, the binders are the binders specified for the respective biomarker in Table 1, or equivalents thereof, such equivalents thereof having the same CDRs, and/or a sequence identity of at least 80%, at least 90% or at least 95%.

**Table 1: . Overview of commercially available antibodies**

| **Biomarker** | **Antibodies** | **Preferred** |
|---|---|---|
| BDNF | • Recombinant Monoclonal Mouse anti-human IgG2b isotype, Clone #BDNF129-13 (Merck KGaA, Darmstadt, Germany) | • Recombinant Monoclonal Mouse anti-human IgG_{2A} Clone # 37129R (R&D Systems, Inc., Minneapolis, USA) |
| | • Recombinant Monoclonal Mouse anti-human IgG2aκ Clone # 1B10 (Life Technologies GmbH, Darmstadt, Germany) | |
| | • Recombinant Monoclonal Mouse anti-human IgG_{2A} Clone # 37129R (R&D Systems, Inc., Minneapolis, USA) | |
| VEGF | • Recombinant Monoclonal Mouse anti-human IgG2aκ Clone # 3F7 (Abnova, Taipei City, Taiwan) | • Recombinant Monoclonal Mouse anti-human IgG_{2B} Clone # 26503 (R&D Systems, Inc., Minneapolis, USA) |
| | • Recombinant Monoclonal Mouse anti-human IgG_{2B} Clone # 26503 (R&D Systems, Inc., Minneapolis, USA) | |
| IL-18 | • Recombinant Monoclonal Mouse anti-human IgG2B Clone # 132029 (Leinco Technologies, Inc., St. Louis, Missouri, USA) | • Recombinant Monoclonal Mouse anti-human I_{G}G1κ Clone # 125-2H (MBL International, Woburn, MA, USA) |
| | • Recombinant Monoclonal Mouse anti-human I_{G}G1κ Clone # 125-2H (MBL International, Woburn, MA, USA) | |
| MCP-1 | • Recombinant Monoclonal Mouse anti-human IgG2aκ Clone # 15D7 (ProteoGenix, Schiltigheim, France) | • Goat anti-human Polyclonal Ig Clone # Poly5360, RRID:AB_2734497 (BioLegend, San Diego, USA) |
| | • Goat anti-human Polyclonal Ig Clone # Poly5360, RRID:AB_2734497 (BioLegend, San Diego, USA) | |
| TGF-b | • Recombinant Monoclonal Mouse anti-human IgG Clone # 9016 (fisher scientific, Schwerte, Germany) | • Recombinant Monoclonal Mouse anti-human IgG1κ Clone # TW7-16B4 (BioLegend, San Diego, USA) |
| | • Recombinant Monoclonal Mouse anti-human IgG1κ Clone # TW7-16B4 (BioLegend, San Diego, USA) | |
| IGF-1 | • Recombinant Monoclonal Mouse anti-human IgG2aκ Clone # SAA0432 (ProteoGenix, Schiltigheim, France) | • Goat anti-human Polyclonal Ig Clone against human IGF-I, Kit E20, CE-label DE/CA40/00809/20 (mediagnost, Reutlingen, Germany) |
| | Goat anti-human Polyclonal Ig Clone against human IGF-I, Kit E20, CE-label DE/CA40/00809/20 (mediagnost, Reutlingen, Germany) | |
| NfL | • Recombinant Monoclonal Mouse anti-human IgG1 Clone # 2G10 (Novus Biologicals, LLC, Centennial, CO, USA) | • Recombinant Monoclonal Mouse anti-human IgG1 against human NfL, clone # UD1 (UmanDiagnostics, Umea, Sweden) |
| | • Recombinant Monoclonal Mouse anti-human IgG1 against human NfL, clone # UD1 (UmanDiagnostics, Umea, Sweden) | |

"Level" refers to either the amount or concentration of a biomarker according to the invention. The concentration of the biomarker according to the invention usually refers to the abundance of a constituent divided by the total volume of a mixture in a sample. The amount of a biomarker according to the invention usually refers to the number of particles or elementary entities in a sample.

The following embodiments are subject of the invention:
1. A method for
   a) diagnosing Alzheimer's disease or
   b) determining the risk of suffering from Alzheimer's disease
   in a subject,
   wherein the method comprises determining the level of at least the biomarkers Neurofilament light Chain (NfL), brain-derived neurotrophic factor (BDNF), and tumor growth factor beta 1 (TGF-beta 1) and in addition determining the level of one or more biomarkers selected from the group comprising interleukin 18 (IL-18), Monocyte chemotactic protein-1 (MCP-1), Insulin-like growth factor (IGF) and Vascular endothelial growth factor (VEGF) in a sample of bodily fluid of said subject,
   calculating a score from the determined biomarker levels, and
   comparing said score with a reference score,
   and wherein said subject is diagnosed with Alzheimer's disease, or said subject is determined as having a risk of suffering from Alzheimer's disease, if the score is above said reference score.
2. The method according to embodiment 1, wherein the biomarkers are in the form of proteins or peptides.
3. The method according to embodiments 1 or 2, wherein the bodily fluid is selected from the group comprising whole blood, serum, plasma, nasal secretion, cerebrospinal fluid (CSF) and saliva, particularly whole blood, serum, plasma, and cerebrospinal fluid (CSF), more particularly whole blood, serum, and plasma, even more particularly plasma.
4. The method according to embodiments 1 to 3, wherein said reference score is a predetermined reference score.
5. The method according embodiments 1 to 4 characterized in that the determination is performed using an immunoassay or mass spectrometry, wherein particularly the immunoassay is an assay selected from the group comprising ELISA, RIA, multiplex immunoassay or immunofluorescence assay, western blot, line assay and dot blot assay.
6. The method according to embodiments 1 to 5, wherein the method comprises determining the level of at least the biomarkers
   a) NfL, BDNF, TGF-beta 1 and IL-18 or
   b) NfL, BDNF, TGF-beta 1 and MCP-1 or
   c) NfL, BDNF, TGF-beta 1 and IGF or
   d) NfL, BDNF, TGF-beta 1 and VEGF,
   particularly NfL, BDNF, TGF-beta 1 and IL-18, or NfL, BDNF, TGF-beta 1 and MCP-1, more particularly NfL, BDNF, TGF-beta 1 and IL-18.
7. The method according to embodiments 1 to 6, wherein the method has a specificity of at least 70%, particularly of at least 80%, more particularly of at least 90%, even more particularly of at least 95 %.
8. The method according to embodiments 1 to 7, wherein the method is used for the stratification of subjects into risks groups.
9. A kit comprising
   one or more binders that bind specifically to NfL, one or more binders that bind specifically to BDNF, and one or more binders that bind specifically to TGF-beta 1, and furthermore comprising one or more binders that bind specifically to a biomarker selected from the group comprising IL-18), MCP-1, IGF and VEGF, particularly IL-18, and
   optionally furthermore comprising instructions on how to use the kit in a method according to any one of embodiments 1 to 8.
10. The kit according to embodiment 9, which is an ELISA, RIA, multiplex immunoassay, immunofluorescence assay, western blot, line assay or dot blot assay.
11. The kit according to embodiments 9 or 10, wherein the binders are selected from the group comprising antibodies, antibody fragments and non-Ig scaffolds, particularly antibodies and antibody fragments.
12. The kit according to embodiments 9 to 11 for use in a method according to embodiments 1 to 8.

### Figure description

### Figure 1

Receiver operating characteristic (ROC) curves with resulting areas under the curve (AUC) for the combination of BDNF, IGF-1, VEGF, TGF-β1, MCP-1, IL-18.

### Figure 2

Receiver operating characteristic (ROC) curves with resulting areas under the curve (AUC) for the combination of BDNF=brain-derived neurotrophic factor, TGF-β1=transforming growth factor β-1, IL-18=interleukin-18, NfL=neurofilament light.

### Figure 3

Receiver operating characteristic (ROC) curves with resulting areas under the curve (AUC) for NfL.

### Examples

### Example 1

The diagnostic value of NfL, BDNF, TGF-β1, and IL-18 for AD was examined in a memory clinic cohort consisting of unstratified patients with a wide variety of accompanying diseases, co-medications and diagnoses as well as healthy controls. This is as opposed to cohorts of related research questions (Jessen F, et al. Alzheimer's Research and Therapy. 2018;10: 15; Weiner MW, Veitch DP, Aisen PS, Beckett LA, Cairns NJ, Cedarbaum J, et al. 2014 Update of the Alzheimer's Disease Neuroimaging Initiative: A review of papers published since its inception. Alzheimer's and Dementia. 2015;11:e1-e120; Rowe CC et al. Neurobiology of Aging. 2010;31: 1275-1283), which mostly only include AD patients strictly screened for accompanying diseases/co-medication and healthy controls, but no mixed cohorts reflecting physicians' everyday clinical work.

Data are compared to the established patent (WO 2015/113995 A1) of six biomarkers BDNF, IGF-1, VEGF, TGF-β1, MCP-1, and IL-18 measured on a conventional ELISA platform.

### Material and methods

### Participants overview

The sample (N=401) consisted of participants of the observational study "Earlier and Differential Diagnosis of Alzheimer's Disease" (EDAD).

Cognitively impaired patients (n=286) were consecutive referrals to the Memory Clinic of the University Department of Geriatric Medicine FELIX PLATTER, Basel, Switzerland. Patients had not been diagnosed before regarding their cognitive impairment. They were required to have obtained at least 20/30 points on the Mini-Mental Status Examination (MMSE Folstein MF, et al. J Psychiatr Res. 1975;12: 189-198). Patients were diagnosed according to the fifth edition of the Diagnostic and Statistical Manual of Mental Disorders (DSM-5 (American Psychiatric Association. Diagnostic and statistical manual of mental disorders (5th ed.). Washington, DC; 2013)). Diagnoses were reached by consensus decisions made by a panel of neurologists, neuropsychologists, psychiatrists, and neuroradiologists taking into consideration clinical, neuropsychological, and imaging data.

Cognitively healthy subjects (n=115) were participants of the Memory Clinic's "Registry of Cognitively Healthy Individuals Interested to Participate in Research". They were required to have an age-, education-, and gender-adjusted z-standard score on the German version of the Montreal Cognitive Assessment (MoCA (Nasreddine ZS, Journal of the American Geriatrics Society. 2005;53: 695-699) >-1.0. Additionally, healthy controls needed to have a mean score of <3.28 in the 7-item "Informant Questionnaire on Cognitive Decline in the Elderly" (Ehrensperger MM, et al. Psychogeriatrics. 2010;22: 91-100), indicating no decrease in cognitive functioning in the last 2 years.

### Blood Sampling

Blood samples were taken by trained nurses and collected in neutral polypropylene 7.5 ml S-Monovette^{®} tubes without additives (Sarstedt, Nümbrecht, Germany) as well as in glass 5-ml BD Vacutainer^{®} tubes without additives (BD Biosciences, NJ, USA). The samples were given time to clot for 60 min while protected from heat, before being centrifuged at room temperature for 10 min at 2000 × g to segregate the serum. The maximum time from taking the blood sample until the freezing of the serum was 120 min. The serum was frozen and stored in polypropylene tubes at -80°C until analysis. Hemolytic, lipemic or icteric samples were excluded.

### Measurement of BDNF, IGF-1, VEGF, TGF-β1, MCP-1, NfL and IL-18 on the ELISA platform

The protein levels of the six biomarkers BDNF, IGF-1, VEGF-A, TGF-β1, MCP-1, and IL-18 were assessed using ELISAs, described in greater detail in Schipke *et al.* (Schipke CG et al. et al. Neurodegener Dis Manag. 2019;9: 193-203.). NfL was assayed according to the manual supplied by UmanDiagnostics. Briefly, the quantifications were performed according to the manufacturer's instructions with the application of the specific sample dilutions (see table 2). These sample dilutions were optimized and established for each biomarker in our laboratory. In addition to the assay-specific protein standards, an internal control was measured within each assay run. Samples from both blood tube types were assayed within the same runs. Assay results were quantified using a microplate reader (Versamax, Molecular Devices, San Jose, CA, USA). The protein concentrations for BDNF, IGF-1, NfL and TGF-β1 are expressed in ng/ml and pg/ml for VEGF, MCP-1, and IL-18.

**Table 2 - Details on biomarkers/assays**

| **Marker / Kit** | **Catalogue No.** | **Manufacturer** | **Range Standar ds** | **Number of Standards** | **Sample Dilution** | **Incubatio n Times** |
|---|---|---|---|---|---|---|
| Human Free **BDNF** Immunoassay | DBD00 SBD00 | R&D (BioTechne) | 62.5 - 4000 pg/ml | 7 + blank | 1:20 | 30min - 2h |
| Insulin-like Growth Factor I **(IGF-I)** (IGFBP-blocked) | E20 | mediagnost | 2 - 50 ng/ml | 5 + blank | 1:21 | 15min - 1h |
| Human **VEGF** Immunoassay | DVE00 SVE00 | R&D (BioTechne) | 31.3 - 2000 pg/ml | 7 + blank | undiluted | 25min - 2h |
| **Total TGF-b1** | 436707 | BioLegend | 7.8 - 500 pg/ml | 7 + blank | 1:200 (final dilution factor) | 10min - 2h |
| Human **MCP-**1/CCL2 | 438807 | BioLegend | 7.8 - 500 pg/ml | 7 + blank | undiluted | 15min - 2h |
| Human **IL-18** ELISA Kit | 7620 | MBL (medical & biological laboratories CO.,LTD.) | 25.6 - 1000 pg/ml | 5 + blank | Undiluted or 1:5 if out of range | 30min - 1h |
| NfL | 20-8002 | UmanDiagnos tics | 0.5 - 40 pg/ml | 7 + blank | 1:4 | 30min - 2h |

### Measurement of BDNF, TGF-β1, IL-18, MCP-1 and NfL on the ELLA platform

The concentrations of the four biomarkers BDNF, TGF-β1, IL-18, MCP-1 and NfL were assessed using the platform Ella (ProteinSimple, San Jose, CA, USA), a fully automated immunoassay workbench system working with a closed cartridge system. The protein concentrations are expressed in pg/ml for all biomarkers.
NfL was quantified using the "Simple Plex assay for the detection of human Neurofilament Light (NfL) in serum, plasma (EDTA/Heparin), and cerebrospinal fluid (CSF)"
(Proteinsimple, San Jose, California, USA). TGF-beta1 was quantified using the "Simple Plex Human TGF-beta 1 Cartridge" and BDNF, VEGF, MCP-1 and IL-18 were quantified simultaneously on a custom-made plate ("Simple Plex BDNF/Free, CCL2/MCP-1, IL18/IL-1F4, VEGF-A").

Regarding buffers, quantifications were performed according to the manufacturer's instructions with the application of the specific sample dilutions (see table 3). These sample dilutions were optimized and established for each biomarker. Two controls of known concentration provided by the manufacturer and an additional control from pooled serum samples were measured within each assay run.

Samples were diluted (see table 3) and then inserted into the cavities present on the Simple Plex cartridges, buffer was inserted into the respective cavities on the cartridges and cartridges were inserted into the Ella machine. Standards were not pipetted separately, but values were provided for each cartridge by the manufacturer. The software provided with the Ella system calculated the concentrations for each analyte in each individual serum sample based on fluorescence intensities (averages values from 3 individual readouts for each sample from 3 individual "glass nano reactors") that are determined using laser-technology. The overall run-time for a cartridge in the Ella system was 90 minutes.

**Table 3: Details on biomarkers/cartridges used in an exemplary study, LLOQ: lower limit of quantification, ULOQ: upper limit of quantification**

| **Product** | **Part number (and Version)** | **Manufacturer** | **LLOQ-ULOQ** | **Sample Dilution** |
|---|---|---|---|---|
| Simple Plex Human **TGF-beta 1** Cartridge | SPCKB-PS001369 (V4) | Proteinsimple (biotechne) | 20.8 - 12684 pg/ml | 1:15 (final dilution factor) |
| Simple Plex Human **NfL** Cartridge | SPCKB-PS002448 (V4) | Proteinsimple (biotechne) | 2.70 - 10290 pg/ml | 1:2 |
| Simple Plex **BDNF**/Free, CCL2/**MCP-1**, **IL18**/IL-1F4, **VEGF-A** Cartridge | SPCKC-PS003429 (V4) | Proteinsimple (biotechne) | BDNF 8.32 - 36000 pg/ml | 1:2 |
| | | | MCP-1 1.52 - 5780 pg/ml | |
| | | | IL-18 0.96 - 3660 pg/ml | |
| | | | VEGF 3.65 - 5570 pg/ml | |

The method described herein using the Simple Plex System is advantageous because a multitude of biomarkers can be tested in parallel but without provoking cross-reactions. Triple or quadruple combinations can be tested on one plate or cartridge.

All seven biomarkers presented in this study can be quantified using the Simple Plex assay. Depending on the biomarker combination to be tested, one or more cartridges have to be provided.

### Statistical Analyses

Group differences were analyzed with t-tests for normally distributed data, Mann-Whitney-tests for data not normally distributed, and Fisher's exact tests for dichotomous data (sex). Demographic and clinical data are given in mean ± standard deviation (SD), and sex is given in percentage.

It is known that NfL in human serum increases with age without necessarily mirroring a pathological process (Khalil M, et al. Nature Communications. 2020;11:812). This finding was replicated in the present data from healthy controls (3.27% increase per year, data not shown). Since significant age differences were found between the groups of healthy controls and patients, NfL values were age-corrected with 3.27% per year.

An algorithm was trained by applying a multiple logistic regression on a defined training set consisting of AD patients and healthy controls. This is a statistical method used to predict a single binary variable (diseased/healthy) using one or more variables. For comparison, this was done for the six biomarkers BDNF, IGF-1, VEGF-A, TGF-β1, MCP-1, and IL-18 measured on ELISA, and the four biomarkers BDNF, TGF-β1, IL-18, and NfL measured on the Ella platform. In detail, the was trained algorithm using a training set containing randomly chosen 75% of AD patients and 75% of healthy controls. The algorithm was then tested on the testing set containing the remaining 25% of AD patients and 25% of healthy controls.

The algorithm was then used for the classification of all patients' data sets.

### Results

### Participant sub-cohorts

Of the n=286 patients, n=119 were diagnosed with a mild (n=27) or major (n=92) neurocognitive disorder due to probable AD according to DSM-5 (see table 4).

Three AD patients and four healthy controls did not have complete biomarker sets and data were excluded from further analysis. This resulted in a sample ofn=116 AD patients and n=111 healthy controls that were included for further analysis. An algorithm using a training set containing randomly chosen 75% of AD patients (n=87) and 75% of healthy controls (n=83) was trained. The algorithm was then tested on the test set containing the remaining 25% of AD patients and 25% of healthy controls.

**Table 4: Demographics and clinical scores of respective participant groups. MoCA-Scores in brackets were transformed from MMSE-scores. AD=Alzheimer's Disease, HC=Healthy Controls, BMI=body mass index, MMSE=mini-mental state examination, MoCA=Montreal cognitive assessment, IQ-Code=Informant Questionnaire on Cognitive Decline in the Elderly)**

| | **AD (n=119)** | **HC (n=115)** | **75% AD (n=87)** | **25% AD (n=29)** | **75% HC (n=83)** | **25% HC (n=28)** | **Testing set Diseased (n=138)** | **Testing set Disease control (n=118)** |
|---|---|---|---|---|---|---|---|---|
| Age | 78.2 ±7.1 | 71.6 ±6.8 | 78.1 ±7.2 | 78.6 ±6.8 | 71.4 ±6.5 | 72.1 ±7.9 | 77.9 ±6.9 | 69.5 ±10.0 |
| Sex (% female) | 48.7 | 52.2 | 47.1 | 55.2 | 53.0 | 46.4 | 46.2 | 38.1 |
| BMI | 26.1 ±4.6 | 25.4 ±5.7 | 25.9 ±4.5 | 25.2 ±3.5 | 26.0 ±4.2 | 25.7 ±4.2 | 25.8 ±4.4 | 26.4 ±4.3 |
| Years of education | 12.6 ±3.2 | 14.0 ±2.9 | 12.7 ±3.2 | 12.8 ±3.3 | 13.9 ±3.0 | 14.2 ±3.1 | 12.7 ±3.3 | 12.8 ±2.9 |
| MMSE | 25.0 ±2.5 | - | 25.0 ±2.5 | 24.6 ±2.9 | - | - | 24.7 ±3.6 | 27.4 ±2.2 |
| MoCA | *(18.4* ±.*8)* | 27.8 ±1.6 | *(18.4* ±*3.8)* | *(18* ±*4.4)* | 27.7 ±1.7 | 28.1 ±1.3 | *(18.3* ±*4.1)* | (22.6 ±*4.1)* |
| IQ-Code | 3.8 ±0.5 | 3.0 ±0.2 | 3.8 ±0.6 | 3.9 ±0.5 | 3.0 ±0.1 | 3.0 ±0.1 | 3.7 ±0.9 | 3.6 ±0.6 |

### Patient characteristics

An overview of participants' characteristics is presented in Table 4 with demographics and clinical scores. The mean MMSE in the AD group was 25±2.5, not significantly differing from its AD sub-cohorts (75% training, 25% testing).

The group of healthy controls had a mean MoCA of 27.8±1.6 and this did not significantly differ from its healthy control (HC) sub-cohorts (75% training, 25% testing). We found significant age differences between AD (77.7±7.2) and HC (70.9±6.8) participants. For this reason, we corrected the NfL values for age, as described above.

### Training and application of an algorithm for the classification of individuals into AD or control

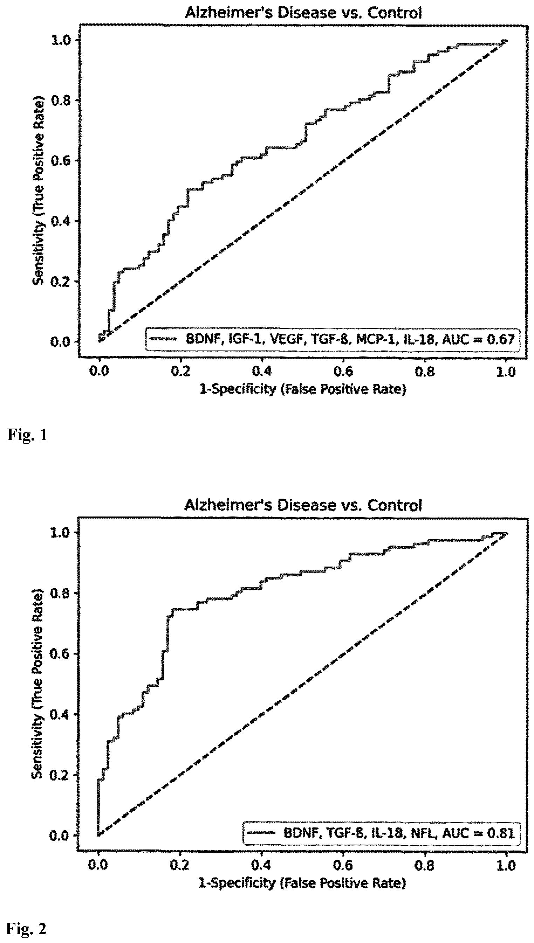

An algorithm to separate healthy controls from AD patients based on the division into groups of 75% and 25% of participants was established. The model was trained with n=83 data sets of healthy controls and n=87 data sets of AD patients. The trained algorithm was then validated with n=28 data sets of controls and n=29 data sets of AD patients. For the six biomarkers BDNF, IGF-1, VEGF-A, TGF-β1, MCP-1, and IL-18 measured on ELISA the AUC was 0,67. For the combined biomarkers BDNF, TGF-β1, IL-18, and NfL measured on Ella the AUC was 0,81. Figures 1 and 2 show areas under the curve (AUC) for both the multiple logistic regression models.

### Example 2 - comparative example No. 1

ROC curves and AUC values were determined using ELISAs as described above for the biomarker panel consisting of BDNF, IGF-1, VEGF, TGF-β, MCP-1, and IL-18.

The results are shown in Figure 1, demonstrating that the biomarker panel according to the present invention allows for a better discrimination, in particular in an "allcomer" population of subjects.

### Example 3 - comparative example No. 2

ROC curves and AUC values were determined using Ella as described above for NfL alone.

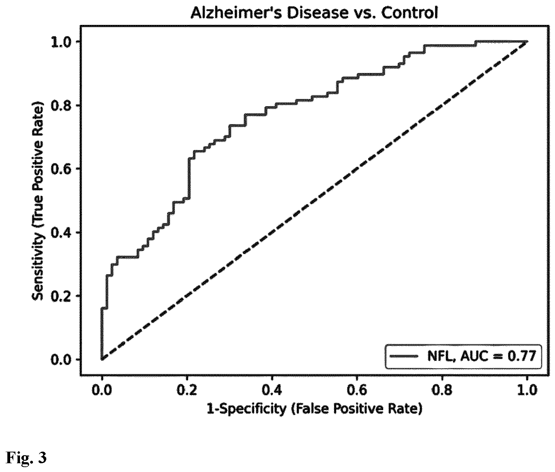

The results are shown in Figure 3, demonstrating that the discrimination with NfL is insufficient and lower than with the biomarker panel of the present invention, in particular in an "allcomer" population of subjects.

### Example 4

In the following, three clinical cases are described:
Case 1:
   An 84-year-old male patient reports to his general practitioner (GP) in company of his wife. His wife describes that he tends to forget appointments recently made. The patients himself describes increasing difficulties in finding places when in public and finding his way home. The GP takes one tube of blood into a plain Sarstedt serum tube and sends it to a medical service laboratory. The laboratory quantifies BDNF, TGF-beta, MCP-1 and NfL in the blood serum. The concentrations are as follows: BDNF 19.2 ng/ml, TGF-beta 36.2 ng/ml, MCP-1 57.2 pg/ml, NfL 95.2 pg/ml. The GP receives those values and enters these values into a software running the algorithm. As output, the software gives the score 0.97, thus indicative of a patient suffering from Alzheimer's disease. Three months later, the patient is diagnosed with mild dementia due to Alzheimer's disease after entering a clinical study with a new compound to treat early Alzheimer's disease.
Case 2:
   A 65-year-old female patient reports to her family doctor and describes increasing difficulties in following conversations and remembering recent activities. The family doctor takes one tube of blood into a plain Sarstedt serum tube and sends it to medical service laboratory. The laboratory quantifies BDNF, TGF-beta, MCP-1 and NfL in the blood serum. The concentrations are as follows: BDNF 8.4 ng/ml, TGF-beta 17.5 ng/ml, MCP-1 40.9 pg/ml, NfL 39.5 pg/ml. The family doctor receives those values and enters these values into a software running the algorithm. As output, the software gives the score 0.54, thus no clear indication can be given with regard to the question whether the patient is suffering from Alzheimer's disease, or not. The patient is advised to come back in 18 months for a blood draw in order to reassess the biomarker values and the score. The patient is advised to see a specialized doctor when symptoms worsen significantly in the meantime.
Case 3:
   A 71-year-old male patient reports to his GP with his daughter. The daughter reports that her father often forgets where his personal belongings are located, such as keys or his phone. She also reports that her father is not remembering all details from recent conversations. The patient himself does not report any difficulties. The GP takes one tube of blood into a plain Sarstedt serum tube and sends it to a medical service laboratory. The laboratory quantifies BDNF, TGF-beta, MCP-1 and NfL in the blood serum. The concentrations are as follows: BDNF 21.6 ng/ml, TGF-beta 38.1 ng/ml, MCP-1 116.1 pg/ml, NfL 29.2 pg/ml.

The family doctor receives those values and enters these values into a software running the algorithm. As output, the software gives the score 0.27, thus not indicative of Alzheimer's disease. Nonetheless neuropsychometric tests are performed at a specialized center. Results from these tests are all within the normal range for the given age-group. Thus, the patient is cognitively healthy and does not suffer from Alzheimer's disease.

### Example 5

The biomarker level of NfL, BDNF, TGF-beta 1, IL-18, MCP-1, IGF and VEGF were compared between healthy individuals and Alzheimer's disease (AD) patients (patient characteristics as described in Example 1) for reference purposes.

### Material and methods

The biomarker level of NfL, BDNF, TGF-beta 1, IL-18, MCP-1, IGF and VEGF were examined in a cohort of Alzheimer's disease patients as well as in healthy controls according to the methods as described in Example 1, see section "blood sampling" & "measurement of BDNF, IGF-1, VEGF, TGF-β1, MCP-1, NfL and IL-18 on the ELLA platform."

### Statistical analysis

The mean level of each of the seven biomarkers was evaluated in each group. No further statistical methods were conducted nor was any algorithm trained or applied to further evaluate the biomarker level.

### Results

The results of the respective measurements are depicted in Table 5.

**Table 5: Comparison of the seven biomarker levels between healthy subject and AD patients.**

| **Biomarker** | **Level in healthy controls** | **Level in AD patients** | **Change in AD** |
|---|---|---|---|
| BDNF | 17.95 ng/ml | 16.7 ng/ml | decrease |
| IGF-1 | 158.3 ng/ | 137.35 ng/ml | decrease |
| VEGF | 568.1 pg/ml | 664.25 pg/ml | increase |
| TGF-beta 1 | 27.85 ng/ ml | 32.35 ng/ml | increase |
| MCP-1 | 123.75 pg/ml | 100.85 pg/ml | decrease |
| IL-18 | 1647.3 pg/ml | 1498.8 pg/ml | decrease |
| NfL | 19.8 pg/ml | 38.65 pg/ ml | increase |

The results show a trend towards decreased BDNF, IGF-1, MCP-1 and IL-18 levels in AD patients, whereas for NfL, VEGF and TGF-beta 1 levels, a trend towards increased levels in said patients could be observed. The findings in the decreased markers mirror the decreased synaptic, metabolic and astrocytic activity in AD pathology whereas the increased markers mirror the increased vascular lesions, altered microglial activity and neurodegenerative processes in AD pathology.

## Claims

1. A method for
a) diagnosing Alzheimer's disease or
b) determining the risk of suffering from Alzheimer's disease
in a subject,
wherein the method comprises determining the level of at least the biomarkers Neurofilament light Chain (NfL), brain-derived neurotrophic factor (BDNF), and tumor growth factor beta 1 (TGF-beta 1) and in addition determining the level of one or more biomarkers selected from the group comprising interleukin 18 (IL-18), Monocyte chemotactic protein-1 (MCP-1), Insulin-like growth factor (IGF) and Vascular endothelial growth factor (VEGF) in a sample of bodily fluid of said subject,
calculating a score from the determined biomarker levels, and
comparing said score with a reference score,
and wherein said subject is diagnosed with Alzheimer's disease, or said subject is determined as having a risk of suffering from Alzheimer's disease, if the score is above said reference score.

2. The method according to claim 1, wherein the biomarkers are in the form of proteins or peptides.

3. The method according to claims 1 or 2, wherein the bodily fluid is selected from the group comprising whole blood, serum, plasma, nasal secretion, cerebrospinal fluid (CSF) and saliva, particularly whole blood, serum, plasma, and cerebrospinal fluid (CSF), more particularly whole blood, serum, and plasma, even more particularly plasma.

4. The method according to claims 1 to 3, wherein said reference score is a predetermined reference score.

5. The method according claims 1 to 4 **characterized in that** the determination is performed using an immunoassay or mass spectrometry, wherein particularly the immunoassay is an assay selected from the group comprising ELISA, RIA, multiplex immunoassay or immunofluorescence assay, western blot, line assay and dot blot assay.

6. The method according to claims 1 to 5, wherein the method comprises determining the level of at least the biomarkers
a) NfL, BDNF, TGF-beta 1 and IL-18 or
b) NfL, BDNF, TGF-beta 1 and MCP-1 or
c) NfL, BDNF, TGF-beta 1 and IGF or
d) NfL, BDNF, TGF-beta 1 and VEGF,
particularly NfL, BDNF, TGF-beta 1 and IL-18, or NfL, BDNF, TGF-beta 1 and MCP-1, more particularly NfL, BDNF, TGF-beta 1 and IL-18.

7. The method according to claims 1 to 6, wherein the method has a specificity of at least 70%, particularly of at least 80%, more particularly of at least 90%, even more particularly of at least 95 %.

8. The method according to claims 1 to 7, wherein the method is used for the stratification of subjects into risks groups.

9. A kit comprising
one or more binders that bind specifically to NfL, one or more binders that bind specifically to BDNF, and one or more binders that bind specifically to TGF-beta 1, and furthermore comprising one or more binders that bind specifically to a biomarker selected from the group comprising IL-18), MCP-1, IGF and VEGF, particularly IL-18, and
optionally furthermore comprising instructions on how to use the kit in a method according to any one of claims 1 to 8.

10. The kit according to claim 9, which is an ELISA, RIA, multiplex immunoassay, immunofluorescence assay, western blot, line assay or dot blot assay.

11. The kit according to claims 9 or 10, wherein the binders are selected from the group comprising antibodies, antibody fragments and non-Ig scaffolds, particularly antibodies and antibody fragments.

12. The kit according to claims 9 to 11 for use in a method according to claims 1 to 8.
